# EUROPEAN PATENT APPLICATION

(11) **EP 4 834 272 A1**
(43) Date of publication of application: **04.11.2026**
(21) Application number: 24911335.8
(22) Date of filing: 26.12.2024
(51) Int. Cl.: A61C 7/00

(54) **METHOD AND APPARATUS FOR OBTAINING TOOTH MODEL, STORAGE MEDIUM, AND ELECTRONIC DEVICE**

(30) Priority: 29.12.2023 CN 202311869829
(71) Applicant: Wuxi Ea Medical Instruments Technologies Limited, Wuxi, Jiangsu 214174 (CN)
(72) Inventor: ZHOU, Bowen, Shanghai 200433 (CN); FENG, Yang, Shanghai 200433 (CN)
(74) Representative: Kraeh, Christian
(86) International application number: PCT/CN2024/142735
(87) International publication number: WO 2025/140414

(57) **Abstract**

Disclosed in the present disclosure are a method and apparatus for obtaining a tooth model, a storage medium, and an electronic device. The method comprises: acquiring first tooth model data of a first tooth of a patient, wherein the first tooth comprises at least one accessory; predicting, on the basis of a neural network model, the first tooth model data to obtain predicted point cloud data, wherein the predicted point cloud data is tooth model data which is predicted on the basis of the neural network model and does not comprise data of a first accessory, and the first accessory is an accessory on the first tooth; determining, according to the predicted point cloud data, a first region of the first tooth model data, wherein the first region comprises an area corresponding to the first accessory; and generating, according to the first region, the first tooth model data, and the predicted point cloud data, second tooth model data, wherein the second tooth model data is configured for indicating the first tooth with the first accessory removed.

## Description

### Cross-References to Related Applications

The present disclosure claims priority to Chinese Patent Application No. 202311869829.0, filed on December 29, 2023, with the State Intellectual Property Office of the People's Republic of China, entitled "A Method and Apparatus for Obtaining Tooth Model, Storage Medium, and Electronic Device", the entire contents of which are incorporated herein by reference.

### Technical Field

The disclosure herein relates to the field of medical technology, and more particularly to a method, apparatus, storage medium and electronic device for obtaining a tooth model.

### Background

Nowadays, in the process of orthodontic treatment, dental accessories are often used to assist in the correction of teeth. Dental accessories are a kind of physical structure bonded to the teeth, which usually play a role in increasing the retention of the orthodontic appliance and assisting the movement of teeth. Correspondingly, when the process of dental correction is completed, the dental accessories need to be removed from the teeth.

At present, in most cases, the attachment removal method is used to remove the accessories on the teeth. Usually, before the attachment removal surgery, a tooth model is built to simulate the state of the teeth before and after the surgery to predict the condition of the teeth after the surgery.

The related art usually obtains a tooth model with accessories, remove the accessories from the tooth model manually, and then uses the pre-trained model to predict the state of the postoperative tooth. However, the tooth model obtained in this way is usually quite different from the real tooth model, and the result is not accurate enough.

Therefore, how to obtain a true and reliable tooth model before grinding off the tooth accessories is an urgent problem to be solved.

### Summary

The present disclosure provides a method, apparatus, storage medium and electronic device for obtaining a tooth model, so as to at least partially solve the above-mentioned problems existing in the related art.

The present disclosure adapts following technical schemes.

The present disclosure provides a method for obtaining a tooth model, including:
obtaining first tooth model data of a first tooth of a patient; the first tooth includes at least one attachment;
predicting the first tooth model data based on a neural network model to obtain predicted point cloud data; the predicted point cloud data is tooth model data without a first attachment predicted by the neural network model, the first attachment is an attachment on the first tooth;
determining a first region of the first tooth model data based on the predicted point cloud data; the first region includes a region corresponding to the first attachment;
generating second tooth model data based on the first region, the first tooth model data and the predicted point cloud data; the second tooth model data is used to represent the first tooth after removing the first attachment.

In some embodiments, the determining the first region of the first tooth model data based on the predicted point cloud data, includes:
obtaining a first facet of the first tooth model data; the first facet is at least one facet in the first tooth model data;
determining whether the first facet belongs to the first region based on distance information between the first facet and each facet corresponding to the predicted point cloud data, the each facet is formed by three adjacent data points in the predicted point cloud data.

In some embodiments, the determining whether the first facet belongs to the first region based on the distance information between the first facet and the each facet of the predicted point cloud data, includes:
determining position information of the first facet;
determining information of a shortest distance between the first facet and the predicted point cloud data based on the position information of the first facet and position information of the each facet corresponding to the predicted point cloud data; and
determining that the first facet does not belong to the first region based on that the information of the shortest distance satisfies a first threshold condition.

In some embodiments, the determining that the first facet does not belong to the first region based on that the information of the shortest distance satisfies the first threshold condition, includes:
determining a second facet in the predicted point cloud data based on the position information of the first facet and the position information of the each facet corresponding to the predicted point cloud data, a distance between the second facet and the first facet satisfies a first condition;
determining that the first facet does not belong to the first region based on that the distance between the first facet and the second facet satisfies a second threshold condition.

In some embodiments, the position information includes coordinate information of a center point of the facet.

In some embodiments, the generating the second tooth model data based on the first region, the first tooth model data and the predicted point cloud data, includes:
determining first-point cloud data based on the first region, the first-point cloud data is other point cloud data in the first tooth model data except the first region;
fusing the first-point cloud data and the predicted point cloud data to generate the second tooth model data.

In some embodiments, the predicting the first tooth model data based on the neural network model to obtain the predicted point cloud data, includes:
obtaining second-point cloud data and third-point cloud data based on the first tooth model data, a data amount of the second-point cloud data is different from a data amount of the third-point cloud data;
performing a feature extraction on the second-point cloud data to obtain second-point cloud feature data;
performing the feature extraction on the third-point cloud data to obtain third-point cloud feature data;
inputting the second-point cloud feature data and the third-point cloud feature data into a prediction subnet of the neural network model to obtain the predicted point cloud data.

In some embodiments, the obtaining the second-point cloud data based on the first tooth model data, includes:
determining first-candidate-point cloud data corresponding to candidate vertices of the first tooth model data;
determining second-candidate-point cloud data in the first tooth model data based on information of a distance between the first-candidate-point cloud data and the first tooth model data;
determining the second-point cloud data based on the first-candidate-point cloud data and the second-candidate-point cloud data.

In some embodiments, the method further includes:
displaying the first tooth model data and/or the second tooth model data based on a content of a display instruction in response to receiving the display instruction.

In some embodiments, before displaying the second tooth model data, the method further includes:
removing the first attachment in the first tooth model data in response to receiving a removal instruction.

In some embodiments, the method further includes:
updating the first region of the first tooth model data in response to receiving an update instruction;
updating the second tooth model data based on the first region after updating, the first tooth model data and the predicted point cloud data; the second tooth model data is used to represent the first tooth after the first attachment is removed.

Some embodiments of the present disclosure provide a method for displaying a tooth model, including:
displaying the second tooth model data, the second tooth model data is obtained by the method for obtaining a tooth model according to any one of above embodiments.

In some embodiments, the method further includes:
displaying the first tooth model data.

In some embodiments, the method further includes:
displaying the first tooth model data in response to a first display instruction input by a user;
   or
displaying the second tooth model data in response to a second display instruction input by a user.

The present disclosure provides an apparatus for obtaining a tooth model, including:
an obtaining module configured to obtain first tooth model data of a first tooth of a patient; the first tooth includes at least one attachment;
a prediction module configured to predict the first tooth model data based on a neural network model to obtain predicted point cloud data; the predicted point cloud data is tooth model data without a first attachment predicted by the neural network model, the first attachment is an attachment on the first tooth;
a determination module configured to determine a first region of the first tooth model data based on the predicted point cloud data; the first region includes a region corresponding to the first attachment;
a generating module configured to generate second tooth model data based on the first region, the first tooth model data and the predicted point cloud data; the second tooth model data is used to represent the first tooth after removing the first attachment.

The present disclosure provides a computer-readable storage medium, storing a computer program, the method described in any one of above embodiments is implemented based on the computer program is executed by a processor.

The present disclosure provides an electronic device including a memory, a processor and a computer program stored in the memory and executable on the processor, the processor implements the method described in any one of above embodiments based on executing the computer program.

At least one of the above technical solutions adopted in the present disclosure can achieve the following beneficial effects.

In the method for obtaining a tooth model provided in the present disclosure, first tooth model data of a first tooth of a patient is obtained; the first tooth includes at least one attachment; based on a neural network model, the first tooth model data is predicted to obtain predicted point cloud data; the predicted point cloud data is the tooth model data predicted by the neural network model excluding the first attachment, and the first attachment is the attachment on the first tooth; based on the predicted point cloud data, a first region of the first tooth model data is determined; the first region includes an area corresponding to the first attachment; based on the first region, the first tooth model data and the predicted point cloud data, second tooth model data is generated; the second tooth model data is used to indicate the first tooth without the first attachment.

When the method for obtaining a tooth model provided by the present disclosure is used to predict the second tooth model data after the attachment is removed from the first tooth model data, the predicted point cloud data of the attachment region after the attachment is removed can be obtained according to the first tooth model data through a neural network model, and the attachment part in the first tooth model data can be removed by comparing with the predicted point cloud data, and finally the first tooth model data after the attachment is removed and the predicted point cloud data are fused to obtain the second tooth model data. Compared with the traditional method, the present method does not require any manual operation during the implementation process, reducing the inaccuracy caused by errors in manual operation. At the same time, the method of obtaining the second tooth model data by fusing the predicted point cloud data with the first tooth model data can not only more accurately restore the shape of the tooth model after the attachment is removed, but also retain the original information of the tooth model to the greatest extent.

### Brief Description of Figures

The drawings described herein are used to provide a further understanding of the present disclosure and constitute a part of the present disclosure. The illustrative embodiments of the present disclosure and their descriptions are used to explain the present disclosure and do not constitute an improper limitation on the present disclosure. In the drawings:
FIG. 1 is a flow chart of a method for obtaining a tooth model in the present disclosure;
FIG. 2 is a schematic diagram of the working process of the neural network model in the present disclosure;
FIG. 3 is a schematic diagram showing the positional relationship between the output of the neural network model and data of the first tooth model in the present disclosure;
FIG. 4 is a schematic diagram of the fusion process of the data of the first tooth model and cloud data of the predicted points in the present disclosure;
FIG. 5 is a schematic diagram of an apparatus for obtaining a tooth model provided by the present disclosure;
FIG. 6 is a schematic diagram of an electronic device corresponding to FIG. 1 provided in the present disclosure.

### Detailed Description

In order to make the purpose, technical solution and advantages of the present disclosure clearer, the technical solution of the present disclosure will be clearly and completely described below in combination with the specific embodiments of the present disclosure and the corresponding drawings. Obviously, the described embodiments are only part of the embodiments of the present disclosure, not all of the embodiments. Based on the embodiments in the present disclosure, all other embodiments obtained by ordinary technicians in this field without making creative work are within the scope of protection of the present disclosure.

The technical solutions provided by various embodiments of the present disclosure are described in detail below in conjunction with the accompanying drawings.

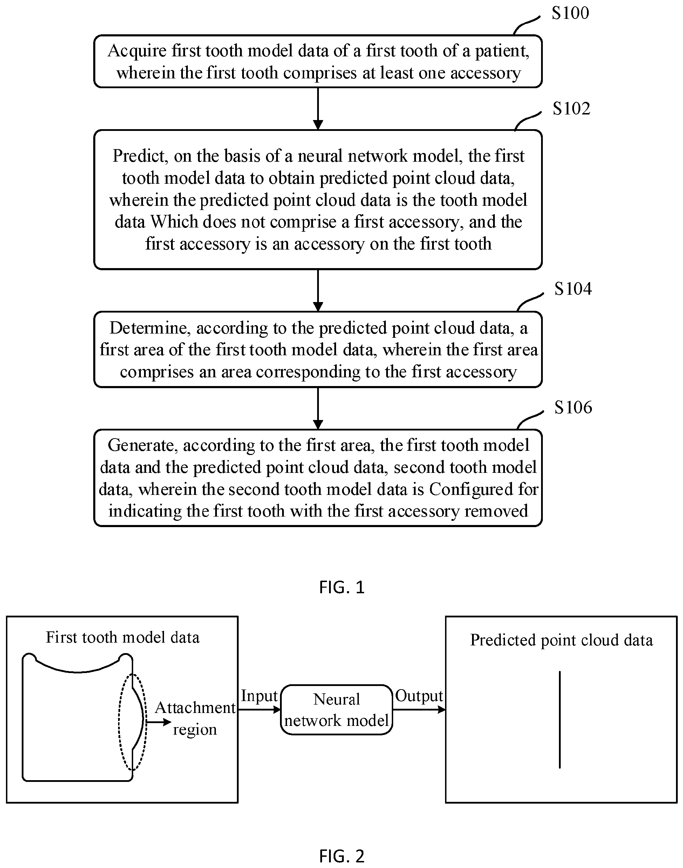

FIG. 1 is a flow chart of a method for obtaining a tooth model provided by the present disclosure, which specifically includes the following steps:
S100: Obtaining the first tooth model data of a first tooth of a patient; the first tooth includes at least one attachment.

All steps in the method for obtaining a tooth model provided in the present disclosure can be implemented by any electronic device with computing functions, such as a terminal, a server, and the like.

The method for obtaining a tooth model provided in the present disclosure is used to simulate in software the state of a tooth with an attachment after the attachment is removed. Therefore, in this step, the first tooth model data of the first tooth of the patient can be first obtained. The first tooth of the patient is a tooth with at least one attachment. The first tooth model data is constructed in a three-dimensional space with every three adjacent vertices forming a face. The vertices can be obtained in a variety of ways, such as oral scanning, etc., and the present disclosure does not impose specific restrictions on this.

S102: Based on the neural network model, predicting the first tooth model data to obtain cloud data of predicted points; the cloud data of the predicted point is the tooth model data predicted by the neural network model excluding the first attachment, and the first attachment is the attachment on the first tooth.

In this step, the first tooth model data obtained in step S100 can be input into the pre-trained neural network model. It can be imagined that when any tooth model is input into the neural network, it can be regarded as inputting the position information of several vertices and the connection relationship between the vertices. In the method for obtaining a tooth model provided in the present disclosure, all tooth models are constructed by forming a face with three adjacent vertices. Therefore, when the connection method between the points is fixed, it can be regarded as inputting a point cloud set into the neural network model.

In the method for obtaining a tooth model provided in the present disclosure, the function of the neural network model is to predict the state of the attachment region in the tooth model after the attachment is removed based on the input point cloud set, and output the cloud data of the predicted points of the tooth model without the attachment.

In addition, in practical applications, one thing that needs to be considered is that for different teeth, the number of vertices in the first tooth model data obtained during oral scanning is different. However, for the neural network model, when the number of vertices in the input point cloud set is fixed, it is easier to adjust the model parameters, and it is also possible to train a neural network model with better prediction results. Therefore, after obtaining the first tooth model data, a point cloud set of a fixed size can be extracted first, and the extracted point cloud set can be used as the input of the neural network model.

Specifically, cloud data of the second point and cloud data of the third-point cloud data can be obtained based on the first tooth model data. The data amounts of the cloud data of the second-point cloud data is different from the data amounts of the cloud data of the third-point cloud data. The feature extraction is performed on the cloud data of the second point to obtain cloud feature data of the second point. The feature extraction is performed on the cloud data of the third point to obtain cloud feature data of the third point cloud. The cloud feature data of the second point and the cloud feature data of the third point are input into the prediction subnet of the neural network model to obtain predicted point cloud data.

Taking obtaining the cloud data of the second point as an example, when obtaining point cloud data of other dimensions, specifically, determining the first-candidate-point cloud data corresponding to the candidate vertices of the first tooth model data, determining the second-candidate-point cloud data in the first tooth model data based on the distance information between the first-candidate-point cloud data and the first tooth model data; determining the second-point cloud data based on the first-candidate-point cloud data and the second-candidate-point cloud data.

In practical applications, one point that needs to be taken into consideration is that when the model has multi-scale input, the model can extract multiple features with different emphases. Therefore, each time the original-point cloud data is constructed according to the first tooth model data, multiple original-point cloud data of different sizes can be constructed, that is, multiple original-point cloud data containing different numbers of vertices, namely the second-point cloud data and the third-point cloud data. Each original-point cloud data is used as input together and input into the neural network model in parallel, so that the neural network model can extract point cloud features of multiple different scales and obtain better output results. Correspondingly, the neural network model can be a model that can receive multi-scale input, such as Point Fractal Network (PF-Net) and other neural network models, and the present disclosure does not impose specific restrictions on this. Among them, the number of original-point cloud data determined, and the number of vertices contained in each original-point cloud data can be preset according to specific needs.

The original-point cloud data can be constructed in a variety of ways, and the present disclosure provides an embodiment for reference. Specifically, for each original-point cloud data, candidate vertices can be selected from the vertices of the first tooth model data to create the original-point cloud data. The minimum distance from each vertex in the first tooth model data that is not in the original-point cloud data to the original-point cloud data is determined. The vertex with the largest minimum distance among the vertices that are not in the original-point cloud data is added to the original-point cloud data, until the number of vertices contained in the original-point cloud data is not less than the number of vertices preset for the original-point cloud data.

A possible implementation method is that the difference between several original-point cloud data determined based on a first tooth model data is that the number of vertices contained is different. Therefore, each original-point cloud data can be constructed in the same way. When constructing an original-point cloud data, initially, the original-point cloud data is empty and does not contain any vertices. First, a vertex can be randomly selected from all the vertices of the first tooth model data and added to the original-point cloud data. Subsequently, among all the remaining vertices, the vertex with the largest minimum distance to the original-point cloud data is selected and added to the original-point cloud data, and this process is repeated until the number of vertices contained in the original-point cloud data is not less than the preset number of vertices of the original-point cloud data. In this way, the original-point cloud data representing the first tooth model data can be obtained.

The minimum distance from a vertex that is not in the original-point cloud data to the original-point cloud data is the minimum value of the distance between the vertex and each vertex in the original-point cloud data. Specifically, when determining the minimum distance from a vertex to the original-point cloud data, the distance between the vertex and each vertex in the original-point cloud data can be determined. The minimum distance is selected from the determined distances as the minimum distance from the vertex to the original-point cloud data.

For example, when there is only one vertex A in the original-point cloud data, the minimum distance from a vertex to the original-point cloud data is the distance between the vertex and vertex A in the original-point cloud data. Therefore, the vertex B with the largest distance from the vertex A in the original-point cloud data among other vertices in the first tooth model data can be added to the original-point cloud data. At this time, there are two vertices in the original-point cloud data, namely vertex A and vertex B. Then at this time, the minimum distance from a vertex that is not in the original-point cloud data to the original-point cloud data is the smaller value between the distance from the vertex to vertex A and the distance from the vertex to vertex B. Among all the vertices that are not in the original-point cloud data, the vertex C with the largest minimum distance to the original-point cloud data is selected and added to the original-point cloud data. The above steps are repeated until the number of vertices in the original-point cloud data meets the preset number of vertices.

Optionally, after the construction of the original-point cloud data is completed, in order to simplify the workload of the neural network model when calculating the data, the original-point cloud data can be normalized. Specifically, the center point of the original-point cloud data can be determined according to the position of each vertex in the original-point cloud data, and the original-point cloud data can be normalized according to the center point.

Among them, the center point of the original-point cloud data can be the average of the position coordinates of each vertex in the original-point cloud data. After determining the center point of the original-point cloud data, all points in the original-point cloud data can be translated so that the center point of the original-point cloud data is located at the origin of the coordinate system. Subsequently, the vertex with the largest distance from the center point in the original-point cloud data can be determined, and all vertices can be normalized using the distance between the vertex and the center point as the normalization coefficient. In other words, the distance between the vertex and the center point is used as the divisor, and the distance between all vertices and the center point is divided by the divisor to obtain the normalized distance between each vertex and the center point, and the position coordinates of each vertex are adjusted accordingly.

S104: Determining a first region of the first tooth model data according to the predicted point cloud data; the first region includes a region corresponding to the first attachment.

According to the predicted point cloud data determined in step S102, a first region in the first tooth model data can be further determined in this step. The first region is a region in the first tooth model data including the first attachment.

When determining the first region, specifically, a first facet of the first tooth model data can be obtained. The first facet is at least one facet in the first tooth model data. Based on the distance information between the first facet and each facet corresponding to the predicted point cloud data, determining whether the first facet belongs to the first region. Each facet is formed by three adjacent data points in the point cloud data.

Among them, when judging whether the first facet belongs to the first region, specifically, the position information of the first facet can be determined. According to the position information of the first facet and the position information of each facet corresponding to the predicted point cloud data, the shortest distance information between the first facet and the predicted point cloud data is determined. When the shortest distance information satisfies the first threshold condition, it is determined that the first facet does not belong to the first region.

In this method, although the first tooth model data and the predicted point cloud data are two different point cloud sets, both are point cloud sets related to the first tooth, and the two are located in the same space and measured using the same coordinate system. In other words, the relative position between the two is the real relative position. Therefore, it can be judged whether the first facet belongs to the first region based on the distance information between the facets.

In the present method, the first region is the region where the attachment included in the first tooth model data is located, that is, the facet in the first region represents the facet of the attachment in the first tooth model data. As described in the above steps, the first tooth model data is a tooth model including the attachment, and the predicted point cloud data is a tooth model excluding the attachment. Based on this, it can be understood that the difference between the first tooth model data and the predicted point cloud data should be the point cloud data of the first region where the attachment is located.

Therefore, it is possible to determine whether the first facet in the first tooth model data belongs to the first region based on the distance information. Specifically, the second facet in the predicted point cloud data can be determined based on the position information of the first facet and the position information of each facet corresponding to the predicted point cloud data, and the distance between the second facet and the first facet satisfies the first condition. When the distance between the first facet and the second facet satisfies the second threshold condition, it is determined that the first facet does not belong to the first region. The position information of a facet can be the coordinate information of the center point of the facet.

Among them, the distance between the second facet and the first facet satisfies the first condition, which can be that the second facet is the facet with the shortest distance to the first facet among all the facets of the predicted point cloud data. The distance between the first facet and the second facet satisfies the second threshold condition, which can be that the distance between the first facet and the second facet is not greater than the specified distance.

By adopting the above method, it is possible to find the facet with the shortest distance to the first facet in the predicted point cloud data, that is, the facet with the highest similarity. When the distance between the two is short enough, that is, not greater than the specified distance, it can be considered that the positions of the two in physical space are repeated. At this time, the first facet and the second facet can be regarded as the same facet in different point cloud sets. Since there is no attachment in the predicted point cloud set, for any first facet in the first tooth model data, as long as there is a facet with a distance not greater than the specified distance in the predicted point cloud set, it can be considered that the first facet is not a facet belonging to the attachment region, that is, it does not belong to the first region. Conversely, for the first facet, when there is no second facet with a distance not greater than the specified distance in the predicted point cloud set, it can be considered that the first facet is a facet representing the attachment and belongs to the first region.

In this way, all the facets in the first tooth model data belonging to the first region can be determined, and then the first region can be determined.

S106: Generating second tooth model data according to the first region, the first tooth model data and the predicted point cloud data; the second tooth model data is used to indicate the first tooth with the first attachment removed.

In this step, the second tooth model data after the attachment is removed can be constructed based on the first tooth model data obtained in step S100, the predicted point cloud data obtained in step S102, and the first region obtained in step S104. Specifically, the first-point cloud data can be determined based on the first region, and the first-point cloud data is the other point cloud data in the first tooth model data except the first region. The first-point cloud data and the predicted point cloud data are fused to generate the second tooth model data.

When constructing the complete second tooth model data with the attachment removed, it is necessary to first remove the attachment in the first tooth model data, and then fuse the predicted point cloud data output by the neural network model, that is, the tooth model without attachment, into the first tooth model data.

In some embodiments, each data point in the first tooth model data is directly collected from real teeth by, for example, three-dimensional oral scanning, and the predicted point cloud data is predicted by a neural network model. Under normal circumstances, the accuracy of the position of each point in the first tooth model data must be higher than that of the predicted point cloud data. Therefore, during the fusion process, for regions in the first tooth model data that are not related to the attachment, this part of the content can be directly retained without using the content in the predicted point cloud data, further improving the accuracy of the reconstructed second tooth model data.

Based on the above idea, the output of the predicted point cloud data can be changed so that the predicted point cloud data only outputs the tooth surface region of the part of the first tooth with the attachment removed.

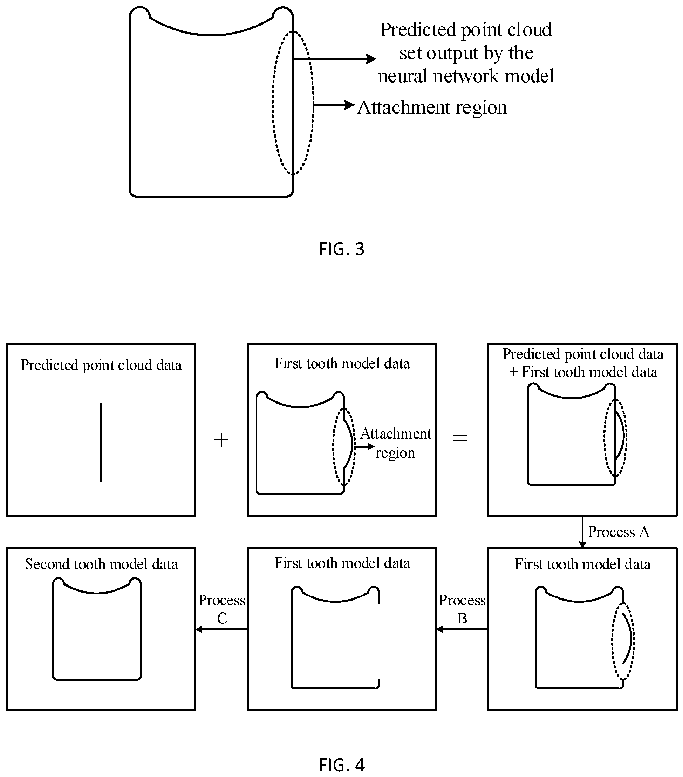

As shown in FIG. 2 and FIG. 3, FIG. 2 is a schematic diagram of the predicted point cloud data output by the neural network model based on a first tooth model data. FIG. 3 is a schematic diagram after the output of the neural network model is restored to the first tooth model data. For ease of understanding, FIG. 2 and FIG. 3 only show simplified views of the point cloud set, and each vertex is not drawn in FIG. 2 and FIG. 3.

In FIG. 2, the arc-shaped protrusion on the right side of the first tooth model data is the attachment, and the part circled by the dotted ellipse in the figure is the attachment region, that is, the attachment and the region around the attachment. After the first tooth model data is input into the neural network model, the neural network model will output a point cloud set of the tooth surface region after the attachment in the attachment region is ground off. In FIG. 2, since the observation angle is from the side of the tooth surface, it is observed as a straight line. When observed from the front of the tooth surface, a plane can actually be observed. FIG. 3 shows a schematic diagram of restoring the point cloud set of the attachment region output by the neural network model to the tooth model after the attachment is ground off. It should be noted that FIG. 3 is only a schematic diagram given in the present disclosure to facilitate the understanding of the model output. In actual applications, the restoration step shown in FIG. 3 does not exist.

At this time, the method of removing the attachment in the first tooth model data is to isolate the attachment in the first tooth model data by comparing the first tooth model data with the predicted point cloud data, and to achieve the purpose of removing the attachment by deleting the free facets in the first tooth model data.

Specifically, as mentioned in step S100 of the present disclosure, in the method for obtaining a tooth model provided by the present disclosure, the connection relationship of each vertex in all tooth models and point cloud sets is that three adjacent vertices form a facet. Therefore, the facet can be used as the smallest unit to perform the attachment removal operation in the first tooth model data. During the output process of the neural network model, the size of the tooth model and the coordinate system in which it is located will not be changed, so the first tooth model data can be compared with the output predicted point cloud data in the same coordinate system.

For each facet in the attachment region in the first tooth model data, the minimum distance from the facet to the predicted point cloud data can be determined. When the minimum distance is not greater than the specified distance, the facet can be deleted from the first tooth model data. In this way, the attachment in the first tooth model data can be isolated, so that the facet of the attachment becomes a free facet. Subsequently, the free facet in the first tooth model data can be deleted, that is, the attachment in the first tooth model data can be deleted. Finally, the first tooth model data after the attachment is removed is fused with the predicted point cloud data to obtain the second tooth model data with the attachment removed.

Among them, the minimum distance from a facet in the first tooth model data to the predicted point cloud data can be the minimum value of the distances between the facet and all facets in the predicted point cloud data. Specifically, when determining the minimum distance from a facet in the first tooth model data to the predicted point cloud data, the center point of each facet in the first tooth model data can be determined based on the vertices forming each facet in the first tooth model data, and the center point of each facet in the predicted point cloud data can be determined based on the vertices forming each facet in the predicted point cloud data. The distance between the center point of each facet in the attachment region of the first tooth model data and the center point of each facet in the predicted point cloud data is determined, and the minimum distance is selected from the determined distances as the minimum distance from the facet to the predicted point cloud data.

For any facet, the average of the position coordinates of the three vertices forming the facet can be used as the center point of the facet. Thus, the distance between any two facets can be determined as the distance between the center points of the two facets. The minimum distance from a facet in the attachment region of the first tooth model data to the predicted point cloud data can be the minimum value of the distance between the center point of the facet and the center point of each facet in the predicted point cloud data.

FIG. 4 shows the process of constructing the second tooth model data after removing the attachment based on the first tooth model data and the predicted point cloud data. As shown in FIG. 4, the first tooth model data and the predicted point cloud data are first placed in the same coordinate system. Subsequently, in process A, each facet in the attachment region of the first tooth model data whose minimum distance from the predicted point cloud data is not greater than the specified distance is deleted to obtain the first tooth model data with the attachment isolated. Subsequently, in process B, the free facet in the first tooth model data can be deleted, that is, the attachment is deleted to obtain the first tooth model data after removing the attachment. Subsequently, in process C, the first tooth model data and the predicted point cloud data can be fused to obtain the second tooth model data after grinding away the attachment.

In some embodiments, after obtaining the second tooth model data, the vicinity of the suture between the first tooth model data and the predicted point cloud data in the second tooth model data may be smoothed to obtain more natural second tooth model data.

When the method for obtaining a tooth model provided by the present disclosure is used to predict the second tooth model data after the attachment is removed from the first tooth model data, the predicted point cloud data of the attachment region after the attachment is removed can be obtained according to the first tooth model data through a neural network model, and the attachment in the first tooth model data can be removed by comparing with the predicted point cloud data, and finally the first tooth model data after the attachment is removed and the predicted point cloud data are fused to obtain the second tooth model data. Compared with the traditional method, the present method does not require any manual operation during the implementation process, reducing the inaccuracy caused by errors in manual operation. At the same time, the method of obtaining the second tooth model data by fusing the predicted point cloud data with the first tooth model data can not only more accurately restore the shape of the tooth model after the attachment is removed, but also retain the original information of the tooth model to the greatest extent.

In addition, in combination with the actual operation of the user during medical use, the first tooth model data and the second tooth model data constructed in the method can be displayed to the user according to demand. Specifically, in response to receiving a display instruction, the first tooth model data and/or the second tooth model data can be displayed according to the content of the display instruction.

Furthermore, after observing the first tooth model data, the user can, according to demand, use the function of removing the attachment to enable the execution subject to further display the second tooth model data to the user. Specifically, before displaying the second tooth model data, the first attachment in the first tooth model data can be removed in response to receiving a removal instruction. Similarly, in the present method, the execution of determining the first region can also be performed according to user demand. Specifically, the first region of the first tooth model data can be updated in response to receiving an update instruction. The second tooth model data can be updated according to the updated first region, the first tooth model data and the predicted point cloud data. The second tooth model data is used to indicate the first tooth with the first attachment removed.

In addition, the neural network model used in the method for obtaining a tooth model provided in the present disclosure can be pre-trained. Specifically, in an attachment grinding operation, a sample tooth model can be obtained based on the state of the tooth before the operation, and a label can be obtained based on the state of the tooth after the operation. The label is a point cloud set of the region with the attachment on the tooth after the attachment is removed. The sample tooth model is input into the neural network model to be trained, and the predicted point cloud data output by the neural network model is determined. The neural network model is trained with the minimum difference between the predicted point cloud data and the label as the optimization goal.

The sample tooth model is a three-dimensional mesh model of a tooth that needs attachment grinding before the operation. The label is a point cloud set of the region with the attachment on the tooth after the attachment is remove. It is worth mentioning that for the sample tooth model, several point cloud sets can also be constructed according to the method given in the present disclosure, replacing the sample tooth model as the input of the neural network model during training, and the present disclosure will not repeat it here.

A possible implementation is also provided below.
1. Point cloud processing of triangular mesh model.

The initial data for the present disclosure is a single tooth in triangulated mesh. It needs to perform some pre-processing to obtain data that can be used as input to the network.

Here are the steps:
A. Obtaining all vertex coordinates (X, Y, Z) from the three-dimensional tooth model M, and denoting the sampling point set as P;
B. Farthest Point Sampling (FPS): Setting the maximum number of sampled point clouds to N, randomly selecting a vertex pt₁ from all vertices of the 3D tooth model M and adding it to the sampling set P, calculating the distance D₁ from all vertices to pt₁ , selecting the vertex farthest from pt₁ as pt₂, and adding it to the set P. Calculating the distance D₂ from all points not in P to pt₂ , combining D₁ and D₂, and selecting the minimum distance from each non-P point to the point of P, denoted as the distance D from non-P to P;
C. During iteration B: selecting the point with the farthest distance from non-P to P, adding it to P, and updating D. This process continues until the size of point set P is N;
D. Performing distance normalization operation on the points in P: The center point of the P(X, Y, Z) point set can be the average of the position coordinates of each point in the P(X, Y, Z) point set, that is, Mean(P(X, Y, Z)). All points in the P(X, Y, Z) point set are translated by P(X, Y, Z) = P(X, Y, Z) - Mean(P(X, Y, Z)), so that the center point of the P(X, Y, Z) point set moves to the origin of the coordinate system (0, 0, 0), where P(X, Y, Z)_ is the point set obtained after the P(X, Y, Z) point set is translated. The distances from all points of P(X, Y, Z)_ to (0, 0, 0) are calculated to obtain the maximum distance D_{MAX}. Then D_{MAX} is used as the normalization coefficient, and P(X, Y, Z)_processed = P(X, Y, Z)_/D_{MAX} is used to normalize the P(X, Y, Z)_ point set;
E. After ABCD processing, the data set Pᵢₙₚᵤₜ before inputting the network is obtained. Since the network has a multi-scale input and output structure, in actual operation, Pᵢₙₚᵤₜ will be subjected to steps B and C multiple times by setting different N according to multi-scale requirements to obtain the multi-scale Pᵢₙₚᵤₜ (N1, N2, N3) set.

2. A special training method for neural network for attachment removal problem.

A possible problem that is usually addressed in a neural network is to predict the shape of the missing part of an object through the residual part of the object, that is, the input data is the incomplete shape point cloud, and the output data is the missing part point cloud. The input data in the present disclosure is a complete tooth point cloud with attachment attached, and the output data is a tooth point cloud in the attachment region after the attachment is removed. A conventional solution is to predict the point cloud of the attachment, and then remove the predicted point cloud to form an input and output mode applicable to the neural network. But the actual result is that the missing part point cloud generated in this way will have a large deviation from the actual point cloud, especially when the amount of data is small. The advantages of the present disclosure are: 1. The original shape style of the attachment is fixed, but the attachment point cloud is predicted and the missing part is created, and the shape of the missing part is different. In this case, letting the neural network select the region by itself is better than auxiliary selection of the region; 2. In most cases, there is no need to specifically predict the attachment region. As long as the overlap of the generated missing point cloud and the input point cloud in a certain direction is calculated, the attachment region can be inferred, and the effective region overlap is higher than 90%; 3. Better use of data information and expand the application field of the neural network model.

3. Fusion of predicted point cloud and residual point cloud and reconstruction of triangular mesh.

This stage mainly removes the attachment on the 3D mesh and fuses the predicted point cloud with the 3D mesh to generate the shape after the attachment are removed. The specific steps are as follows:
A. Calculating the center point coordinates Center(X, Y, Z) of the facet in the three-dimensional mesh model (i.e., tooth model), and calculating the shortest distance Dₙₑₐᵣₑₛₜ from Center(X, Y, Z) to the predicted point cloud set Output(X, Y, Z) generated by the neural network;
B. When Dₙₑₐᵣₑₛₜ is less than a certain threshold Thr, the corresponding facets on the tooth model are removed, so that the facets of the attachment on the tooth model become free facets. At the same time, the free facets in the tooth model are deleted to obtain the tooth model M_{crop} with the attachment removed;
C. The triangulated meshed Output point cloud is stitched with M_{crop} to form the final tooth model M_{new} after grinding;
D. Smoothing 2 to 3 neighborhoods around the stitching region of M_{new}.

After A, B, C, and D above, the final triangular mesh model M_{new} of the tooth after grinding is obtained. Here, in order to obtain a more robust and accurate attachment region, operation B can also be integrated into the method of predicting the attachment region to comprehensively evaluate the final target region.

4. Neural network structure and parameter setting.

The training method of the neural network can refer to the existing training methods. Generating the required predicted point cloud in a multi-scale manner, and optimizing the prediction accuracy through adversarial loss and distance loss. A possible training parameter and optimizer can be: input data scale: 2048×3, 1024×3, 512×3; output scale: 256×3, 128×3, 64×3; batch size: 24; Adam optimizer; learning rate = 0.0001, etc.

Accordingly, the present disclosure also provides a method for displaying a tooth model, including:
displaying second tooth model data, where the second tooth model data is obtained by the method for obtaining a tooth model provided by the present disclosure. In addition, the method may also display the first tooth model data. Specifically, the first tooth model data may be displayed in response to a first display instruction input by a user, or the second tooth model data may be displayed in response to a second display instruction input by a user.

The first display instruction and the second display instruction may be generated based on the user's operation, for example, the user opens the corresponding software to indicate the display of the first tooth model, or opens the corresponding function in the corresponding patient case. The function may be determined based on actual needs and is not limited here. The display instruction method may also refer to the display instruction or update instruction in the above embodiment.

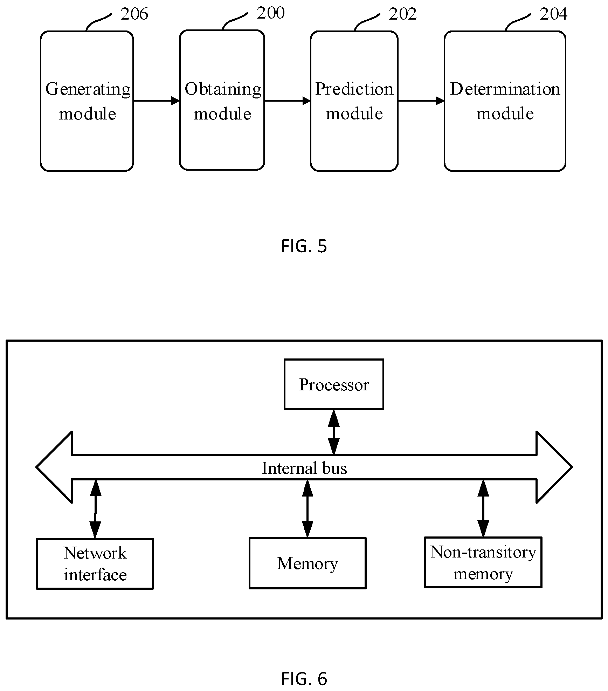

The above is the method for obtaining a tooth model provided by the present disclosure. Based on the same idea, the present disclosure also provides a corresponding apparatus for obtaining a tooth model, as shown in FIG. 5.

FIG. 5 is a schematic diagram of an apparatus for obtaining a tooth model provided by the present disclosure, which specifically includes:
a obtaining module 200 configured to obtain first tooth model data of a first tooth of a patient; the first tooth includes at least one attachment;
a prediction module 202 configured to predict the first tooth model data based on a neural network model to obtain predicted point cloud data; the predicted point cloud data is the tooth model data predicted by the neural network model excluding the first attachment, and the first attachment is an attachment on the first tooth;
a determination module 204 configured to determine a first region of the first tooth model data according to the predicted point cloud data; the first region includes a region corresponding to the first attachment;
a generating module 206 configured to generate second tooth model data according to the first region, the first tooth model data and the predicted point cloud data; the second tooth model data is used to indicate the first tooth with the first attachment removed.

Optionally, the determination module 204 is specifically configured to obtain a first facet of the first tooth model data; the first facet is at least one facet in the first tooth model data; and determine whether the first facet belongs to the first region based on distance information between the first facet and each facet corresponding to the predicted point cloud data. Each facet includes three adjacent data points in the point cloud data.

Optionally, the determination module 204 is specifically configured to determine the position information of the first facet, determine the shortest distance information between the first facet and the predicted point cloud data based on the position information of the first facet and the position information of each facet corresponding to the predicted point cloud data, and determine that the first facet does not belong to the first region based on the shortest distance information satisfies a first threshold condition.

Optionally, the determination module 204 is specifically configured to determine a second facet in the predicted point cloud data based on the position information of the first facet and the position information of each facet corresponding to the predicted point cloud data, and the distance between the second facet and the first facet satisfies a first condition; determine that the first facet does not belong to the first region based on the distance between the first facet and the second facet satisfies a second threshold condition.

Optionally, the position information includes coordinate information of the center point of the facet.

Optionally, the generation module 206 is specifically configured to determine first-point cloud data based on the first region, where the first-point cloud data is other point cloud data in the first tooth model data except the first region; and fuse the first-point cloud data and the predicted point cloud data to generate second tooth model data.

Optionally, the prediction module 202 is specifically configured to obtain second-point cloud data and third-point cloud data based on the first tooth model data, the data amount of the second-point cloud data is different from the data amount of the third-point cloud data; perform feature extraction on the second-point cloud data to obtain second-point cloud feature data, perform feature extraction on the third-point cloud data to obtain third-point cloud feature data, input the second-point cloud feature data and the third-point cloud feature data into the prediction subnet of the neural network model to obtain predicted point cloud data.

Optionally, the prediction module 202 is specifically configured to determine first-candidate-point cloud data corresponding to candidate vertices of the first tooth model data, determine second-candidate-point cloud data in the first tooth model data based on the first-candidate-point cloud data and the distance information and the first tooth model data, and determine second-point cloud data based on the first-candidate-point cloud data and the second-candidate-point cloud data.

Optionally, the apparatus further includes a first response module 208, which is specifically configured to, in response to receiving a display instruction, display the first tooth model data and/or the second tooth model data according to the content of the display instruction.

Optionally, the apparatus further includes a second response module 210, which is specifically configured to remove the first attachment in the first tooth model data in response to receiving a removal instruction.

Optionally, the apparatus also includes a third response module 212, which is specifically configured to update the first region of the first tooth model data in response to receiving an update instruction, update the second tooth model data according to the updated first region, the first tooth model data and the predicted point cloud data; the second tooth model data is used to indicate the removal of the first tooth of the first attachment.

The present disclosure also provides a computer-readable storage medium, which stores a computer program. The computer program can be used to execute the method for obtaining a tooth model provided in FIG. 1 above.

The present disclosure also provides a schematic structural diagram of an electronic device as shown in FIG. 6. As shown in FIG. 6, at the hardware level, the electronic device includes a processor, an internal bus, a network interface, a memory, and a non-volatile memory, and of course may also include hardware required for other services. The processor reads the corresponding computer program from the non-volatile memory into the memory and then runs it to implement the method for obtaining a tooth model and the display method described in FIG. 1 above. Of course, in addition to software implementations, the present disclosure does not exclude other implementations, such as logic devices or a combination of software and hardware, etc., that is, the execution subject of the following processing flow is not limited to each logic unit, but can also be hardware or logic devices.

In a typical configuration, a computing device includes one or more processors (CPU), input/output interfaces, network interfaces, and memory.

The memory may include non-permanent storage in a computer-readable medium, random access memory (RAM) and/or non-volatile memory in the form of read-only memory (ROM) or flash RAM. The memory is an example of a computer-readable medium.

The memory may also include a program tool (or utility) having a set (at least one) of program modules, such program modules including but not limited to: an operating system, one or more application programs, other program modules and program data, each of which or some combination may include the implementation of a network environment.

The processor executes various functional applications and data processing, such as the method in the above embodiment, by running the computer program stored in the memory.

The electronic device can also communicate with one or more external devices. Such communication can be performed through an input/output (I/O) interface. In addition, the electronic device for generating the model can also communicate with one or more networks (such as a local area network (LAN), a wide area network (WAN) and/or a public network, such as the Internet) through a network adapter. The network adapter communicates with other modules of the electronic device through a bus. It should be understood that, although not shown in the figure, other hardware and/or software modules can be used in conjunction with the electronic device, including but not limited to: microcode, device drivers, redundant processors, external disk drive arrays, RAID (disk array) systems, tape drives, and data backup storage systems, etc.

The input and/or output device may include a scanning device, a camera interface, an input device (e.g., a mouse, a keyboard, etc.), a display device (e.g., a monitor), a printer, and/or one or more other input devices. The input/output interface may receive executable instructions and/or data, which may be stored in a data storage device (e.g., a memory), for example, may be used to receive a generated facet centerline and store the generated facet centerline.

In some embodiments, the scanning device can be configured to scan one or more physical dental models of the patient's dentition. In one or more embodiments, the scanning device can be configured to directly scan the patient's dentition and/or dental appliances. The scanning device can be configured to input data into a computing device.

In some embodiments, the camera interface can receive input from an imaging device (e.g., a 2D or 3D imaging device), such as a digital camera, a printed photo scanner, and/or other suitable imaging device. For example, the input from the imaging device can be stored in a memory.

The processor can execute instructions to provide a display of a tooth model, a dental treatment position, a treatment plan, etc. on a display. For example, the computing device can be configured to allow a treatment professional or other user to input a treatment goal. The received input can be sent to the processor as data and/or can be stored in a memory.

Such connectivity may allow for input and/or output of data and/or instructions and other types of information. Some embodiments may be distributed among various computing devices within one or more networks and used to collect, calculate and/or analyze any of the methods mentioned herein.

It should be noted that although several units/modules or sub-units/modules of the electronic device are mentioned in the above detailed description, this division is merely exemplary and not mandatory. In fact, according to the embodiments of the present disclosure, the features and functions of two or more units/modules described above can be embodied in one unit/module. Conversely, the features and functions of one unit/module described above can be further divided into multiple units/modules to be embodied.

Some embodiments provide a computer-readable storage medium on which a computer program is stored. When the computer program is executed by a processor, the method in the above embodiment is implemented.

The readable storage medium may include but is not limited to: a portable disk, a hard disk, a random access memory, a read-only memory, an erasable programmable read-only memory, an optical storage device, a magnetic storage device or any suitable combination of the above.

In a possible implementation manner, the present disclosure may also be implemented in the form of a program product, which includes program code. When the program product runs on a terminal device, the program code is used to enable the terminal device to execute the steps in the above embodiments.

Among them, the program code for executing the present disclosure can be written in any combination of one or more programming languages, and the program code can be executed completely on the user device, partially on the user device, as an independent software package, partially on the user device and partially on a remote device, or completely on the remote device.

Each embodiment in the present disclosure is described in a progressive manner, and the same or similar parts between the embodiments can be referred to each other, and each embodiment focuses on the differences from other embodiments. In particular, for the system embodiment, since it is basically similar to the method embodiment, the description is relatively simple, and the relevant parts can be referred to the partial description of the method embodiment.

The above is only an embodiment of the present disclosure and is not intended to limit the present disclosure. For those skilled in the art, the present disclosure may have various changes and variations. Any modification, equivalent replacement, improvement, etc. made within the spirit and principle of the present disclosure should be included in the scope of the claims of the present disclosure.

## Claims

1. A method for obtaining a tooth model, comprising:
obtaining first tooth model data of a first tooth of a patient; wherein the first tooth comprises at least one attachment;
predicting the first tooth model data based on a neural network model to obtain predicted point cloud data; wherein the predicted point cloud data is tooth model data without a first attachment predicted by the neural network model, the first attachment is an attachment on the first tooth;
determining a first region of the first tooth model data based on the predicted point cloud data; wherein the first region comprises a region corresponding to the first attachment;
generating second tooth model data based on the first region, the first tooth model data and the predicted point cloud data; wherein the second tooth model data is used to represent the first tooth after removing the first attachment.

2. The method according to claim 1, wherein the determining the first region of the first tooth model data based on the predicted point cloud data, comprises:
obtaining a first facet of the first tooth model data; wherein the first facet is at least one facet in the first tooth model data;
determining whether the first facet belongs to the first region based on distance information between the first facet and each facet corresponding to the predicted point cloud data, wherein the each facet is formed by three adjacent data points in the predicted point cloud data.

3. The method according to claim 2, wherein the determining whether the first facet belongs to the first region based on the distance information between the first facet and the each facet of the predicted point cloud data, comprises:
determining position information of the first facet;
determining information of a shortest distance between the first facet and the predicted point cloud data based on the position information of the first facet and position information of the each facet corresponding to the predicted point cloud data; and
determining that the first facet does not belong to the first region based on that the information of the shortest distance satisfies a first threshold condition.

4. The method according to claim 3, wherein the determining that the first facet does not belong to the first region based on that the information of the shortest distance satisfies the first threshold condition, comprises:
determining a second facet in the predicted point cloud data based on the position information of the first facet and the position information of the each facet corresponding to the predicted point cloud data, wherein a distance between the second facet and the first facet satisfies a first condition;
determining that the first facet does not belong to the first region based on that the distance between the first facet and the second facet satisfies a second threshold condition.

5. The method according to any one of claims 3 to 4, wherein the position information comprises coordinate information of a center point of the facet.

6. The method according to claim 1, wherein the generating the second tooth model data based on the first region, the first tooth model data and the predicted point cloud data, comprises:
determining first-point cloud data based on the first region, wherein the first-point cloud data is other point cloud data in the first tooth model data except the first region;
fusing the first-point cloud data and the predicted point cloud data to generate the second tooth model data.

7. The method according to claim 1, wherein the predicting the first tooth model data based on the neural network model to obtain the predicted point cloud data, comprises:
obtaining second-point cloud data and third-point cloud data based on the first tooth model data, wherein a data amount of the second-point cloud data is different from a data amount of the third-point cloud data;
performing a feature extraction on the second-point cloud data to obtain second-point cloud feature data;
performing the feature extraction on the third-point cloud data to obtain third-point cloud feature data;
inputting the second-point cloud feature data and the third-point cloud feature data into a prediction subnet of the neural network model to obtain the predicted point cloud data.

8. The method according to claim 7, wherein the obtaining the second-point cloud data based on the first tooth model data, comprises:
determining first-candidate-point cloud data corresponding to candidate vertices of the first tooth model data;
determining second-candidate-point cloud data in the first tooth model data based on information of a distance between the first-candidate-point cloud data and the first tooth model data;
determining the second-point cloud data based on the first-candidate-point cloud data and the second-candidate-point cloud data.

9. The method according to claim 1, further comprising:
displaying the first tooth model data and/or the second tooth model data based on a content of a display instruction in response to receiving the display instruction.

10. The method according to claim 9, wherein before displaying the second tooth model data, the method further comprises:
removing the first attachment in the first tooth model data in response to receiving a removal instruction.

11. The method according to claim 1, further comprising:
updating the first region of the first tooth model data in response to receiving an update instruction;
updating the second tooth model data based on the first region after updating, the first tooth model data and the predicted point cloud data; wherein the second tooth model data is used to represent the first tooth after the first attachment is removed.

12. A method for displaying a tooth model, comprising:
displaying the second tooth model data, wherein the second tooth model data is obtained by the method for obtaining a tooth model according to any one of claims 1 to 11.

13. The method according to claim 12, further comprising:
displaying the first tooth model data.

14. The method according to claim 12 or 13, further comprising:
displaying the first tooth model data in response to a first display instruction input by a user;
or
displaying the second tooth model data in response to a second display instruction input by a user.

15. An apparatus for obtaining a tooth model, comprising:
an obtaining module configured to obtain first tooth model data of a first tooth of a patient; wherein the first tooth comprises at least one attachment;
a prediction module configured to predict the first tooth model data based on a neural network model to obtain predicted point cloud data; wherein the predicted point cloud data is tooth model data without a first attachment predicted by the neural network model, the first attachment is an attachment on the first tooth;
a determination module configured to determine a first region of the first tooth model data based on the predicted point cloud data; wherein the first region comprises a region corresponding to the first attachment;
a generating module configured to generate second tooth model data based on the first region, the first tooth model data and the predicted point cloud data; wherein the second tooth model data is used to represent the first tooth after removing the first attachment.

16. A computer-readable storage medium, storing a computer program, wherein the method described in any one of claims 1 to 14 is implemented based on the computer program is executed by a processor.

17. An electronic device comprising a memory, a processor and a computer program stored in the memory and executable on the processor, wherein the processor implements the method described in any one of claims 1 to 14 based on executing the computer program.
